# FASCICULE DE BREVET EUROPEEN

(11) **EP 3 365 145 B1**
(45) Date de publication et mention de la délivrance du brevet: **14.08.2019**
(21) Numéro de dépôt: 16798241.2
(22) Date de dépôt: 18.10.2016
(51) Int. Cl.: B25J 21/00, A61L 27/06, B01J 19/00

(54) **ENCEINTE ETANCHE DE POLYMERISATION**
ABGEDICHTETE POLYMERISATIONSKAMMER
SEALED POLYMERISATION CHAMBER

(30) Priorité: 22.10.2015 FR 1560111
(43) Date de publication de la demande: 29.08.2018
(73) Titulaire: Les Laboratoires Osteal Medical, 95957 Roissy Aeroport CDG (FR)
(72) Inventeur: BOUCHOT, Daniel, 77186 Noisiel (FR); DE LAMBERT, Bertrand, 60300 Senlis (FR); TISSERAND, Thierry, 60128 Plailly (FR); PORQUEDDU, Cyril, 95270 Chaumontel (FR)
(74) Mandataire: CAPRI
(86) Numéro de dépôt international: PCT/FR2016/052685
(87) Numéro de publication internationale: WO 2017/068275

(56) Documents cités:
- EP-A2- 2 032 663
- US-A1- 2008 187 415
- US-A1- 2014 196 411

## Description

La présente invention concerne une enceinte étanche contenant un gaz inerte, tel que de l'argon, cette enceinte comprenant un sas d'entrée. En général, de telles enceintes comprennent une paroi avant vitrée munie d'une ou de plusieurs gant(s) de manipulation permettant à une personne de manipuler des objets ou des dispositifs disposés à l'intérieur de l'enceinte étanche. On peut notamment traiter des objets en leur faisant subir toute sorte de procédés qui nécessitent une atmosphère de gaz inerte. L'argon est en général utilisé, mais d'autres gaz inertes peuvent également être employés.

Dans l'art antérieur, on connaît le document EP2032663 qui décrit un procédé de greffage de polymère bioactif sur un matériau prothétique en titane ou alliage de titane. Ce procédé préconise trois étapes successives, à savoir :
- la génération d'espèces actives donneuses de radicaux libres à la surface du matériau prothétique,
- la génération de radicalaire à la surface du matériau prothétique par réactions thermiques, et
- la mise en présence du matériau prothétique avec au moins un monomère porteur d'une fonction permettant une polymérisation radicalaire. La polymérisation radicalaire dudit monomère permettant la formation d'un polymère bioactif en l'absence d'oxygène.

Par conséquent, le procédé de ce document EP2032663 est plus particulièrement orienté sur les réactions et interactions physiques ou chimiques permettant de synthétiser le polymère bioactif directement à partir de la surface d'un implant. Cela donne au polymère bioactif la caractéristique d'être greffée de manière permanente sur l'implant. Le polymère bioactif est de préférence du PolyNaSS et le type d'oxydation de l'implant est de préférence une oxydation chimique.

Bien que le procédé du document EP2032663 trace les grandes lignes à suivre pour réaliser le procédé de greffage en laboratoire à petite échelle, il ne donne absolument aucune indication quant à l'application de ce procédé de greffage de manière industrielle à grande échelle.

La présente invention a précisément pour but d'utiliser ce procédé de greffage de polymère bioactif de manière industrielle à grande échelle. En d'autres termes, il s'agit de mettre en oeuvre ce procédé de greffage dans un milieu industriel afin de traiter de grandes séries d'implants à la fois. Alors que l'oxydation chimique est mise en avant dans le procédé de greffage du document EP2032663, il s'est rapidement avéré que ce type d'oxydation (chimique) est tout à fait inapproprié, voire dangereux, dans un environnement industriel. La présente invention s'est ainsi tournée vers l'oxydation anodique, qui n'est pas mentionnée dans le document US2014/196411 décrit une enceinte étanche avec un sas d'entrée et des moyens de manipulation d'objets.

Afin d'améliorer l'industrialisation de ce procédé de greffage de polymère bioactif, la présente invention propose une enceinte étanche contenant un gaz inerte, tel que de l'argon, l'enceinte étanche comprenant un sas d'entrée, caractérisée en ce qu'elle contient en outre une station de polymérisation pour greffer un polymère bioactif, tel que du PolyNaSS, sur un implant, la station de polymérisation comprenant :
- une cuve destinée à contenir un monomère,
- des moyens catalyseurs agissant sur le monomère de la cuve pour accélérer la polymérisation,
- un ascenseur comprenant un chariot déplaçable verticalement au-dessus de la cuve pour plonger/extraire les implants dans/hors de la cuve.

L'enceinte étanche de l'invention constitue ainsi un des équipements industriels nécessaires pour la mise en oeuvre du procédé de greffage de polymère bioactif à la surface d'un implant.

Selon une forme de réalisation avantageuse, les moyens catalyseurs comprennent un bac de bain-marie dans lequel est disposée la cuve. En variante, les moyens catalyseurs comprennent au moins une source de rayonnement UV qui irradie le contenu de la cuve. Avantageusement, la cuve peut comprendre une paroi de fond et au moins une paroi latérale, au moins une paroi parmi la paroi de fond et ladite au moins une paroi latérale est réfléchissante pour le rayonnement UV. Selon un autre aspect avantageux, la cuve comprend une paroi de fond et au moins une paroi latérale, au moins une paroi parmi la paroi de fond et ladite au moins une paroi latérale présente une forme elliptique. Selon une forme de réalisation avantageuse, la cuve comprend une paroi de fond et une paroi latérale elliptique pourvue d'au moins deux sources de rayonnement UV disposées sur le grand axe de l'ellipse. Selon une autre forme de réalisation pratique, la cuve comprend une paroi de fond et une paroi latérale formant plusieurs cavités de réflexion, avantageusement elliptiques, pourvues chacune d'une source de rayonnement UV. La forme elliptique ou ellipsoïdale de la cuve permet de réfléchir de manière optimale les rayonnements UV émis par la ou les source(s).

Selon une autre forme de réalisation pratique, la cuve comprend une paroi de fond et au moins une paroi latérale, au moins une paroi parmi la paroi de fond et ladite au moins une paroi latérale comprend un panneau LED UV. En variante, au moins une des parois peut être formée par un panneau LED UV. On peut même imaginer un mode de réalisation dans lequel toutes les parois de la cuve sont formées par des panneaux LED UV raccordés les uns aux autres.

Selon l'invention, l'ascenseur comprend un chariot mobile verticalement pourvu de moyens de montage aptes à recevoir des implants ou une structure de support d'implants. Avantageusement, les moyens de montage comprennent au moins une glissière dans laquelle des moyens de fixation de la structure de support sont insérables par coulissement. De préférence, le chariot mobile comprend une série de glissières parallèles formant des ouvertures d'accès sur une face frontale tournée vers les gants de manipulation. D'autre part, l'ascenseur peut comprendre un couvercle mobile verticalement pour couvrir la cuve en position basse.

Selon un autre mode de réalisation particulièrement avantageux, le chariot comprend au moins une roue horizontale qui est montée rotative autour d'une tige axiale verticale sur laquelle est monté l'implant ou une structure de support d'implants, de sorte que l'implant tourne sur la tige axiale verticale dans le monomère de la cuve. Ainsi, l'implant n'est pas maintenu de manière statique à l'intérieur de la cuve, mais se déplace à l'intérieur du monomère de manière à exposer toutes ses surfaces de manière homogène au rayonnement UV.

Avantageusement, la roue horizontale se déplace selon une trajectoire circulaire à l'intérieur d'une couronne de révolution autour une roue d'entrainement centrale, de sorte que l'implant tourne sur la tige axiale verticale qui se déplace selon la trajectoire circulaire dans le monomère de la cuve. Le mouvement de l'implant dans le monomère est ainsi complexe, puisqu'il résulte de la combinaison d'une rotation autour de la tige axiale verticale et d'une révolution autour de la roue d'entraînement centrale. On garantit ainsi que l'implant est exposé de manière homogène au rayonnement UV.

Selon une caractéristique additionnelle intéressante, la roue d'entrainement centrale est pourvue d'un moyeu réfléchissant qui plonge dans la cuve. Les implants se déplacent ainsi entre la paroi externe de la cuve munie des sources UV et le moyeu central axial qui permet de réfléchir les rayonnements UV.

L'esprit de la présente invention réside dans le fait que cette enceinte étanche soit dotée d'un équipement industriel optimal pour réaliser la polymérisation du polymère bioactif sur des implants de manière industrielle, c'est-à-dire de manière reproductible, contrôlée, fiable, rapide et peu onéreuse.

L'invention sera maintenant plus amplement décrite en référence aux dessins joints donnant plusieurs modes de réalisation de l'invention.

Sur les figures :
La figure 1 est une vue très schématique en bloc diagramme représentant les différentes étapes du procédé de greffage selon l'invention,
La figure 2a est une vue en perspective d'un élément de support d'implant selon l'invention,
La figure 2b est une vue de l'élément de support d'implants de la figure 2a avec des implants montés dessus,
La figure 2c est une vue en coupe transversale verticale agrandie à travers un implant monté sur une broche de l'élément de support d'implants des figures 2a et 2b,
La figure 3 est une vue en perspective d'une platine de montage d'éléments de support d'implants qui a déjà reçu trois éléments de supports d'implants et qui est prêt à en recevoir un quatrième,
La figure 4 est une vue en perspective d'un récipient rempli de gaz inerte dans lequel est déposée la platine de montage avec ses éléments de support d'implants,
La figure 5a est une vue en perspective schématique d'une enceinte mise en oeuvre pour l'étape de polymérisation,
La figure 5b est une vue en perspective agrandie d'un détail de la figure 5a,
La figure 6 est une vue en perspective d'une plaque de montage dans laquelle les éléments de support d'implants sont reçus par coulissement,
La figure 7 est une vue en plan schématique de la station de polymérisation disposée à l'intérieur de l'enceinte de polymérisation,
Les figures 8a à 8e représentent différents modes de réalisation d'une cuve de polymérisation contenant le monomère,
Les figures 9a et 9b sont des vues en perspective d'un autre mode de réalisation de la station de polymérisation,
Les figures 10a et 10b représentent deux variantes de cuves recevant le dispositif des figures 9a et 9b,
La figure 11 est une vue en perspective d'un panier de lavage selon l'invention,
La figure 12a représente un étrier pour l'anodisation de deux implants fémoraux de hanche,
La figure 12b représente un implant fémoral de hanche disposé dans un récipient rempli de gaz inerte,
La figure 12c représente une réglette sur laquelle sont montés les implants fémoraux de hanche,
La figure 12d représente deux réglettes engagées dans des rails de montage de l'ascenseur de la station de polymérisation, et
La figure 12e représente un casier de lavage pour des implants fémoraux de hanche.

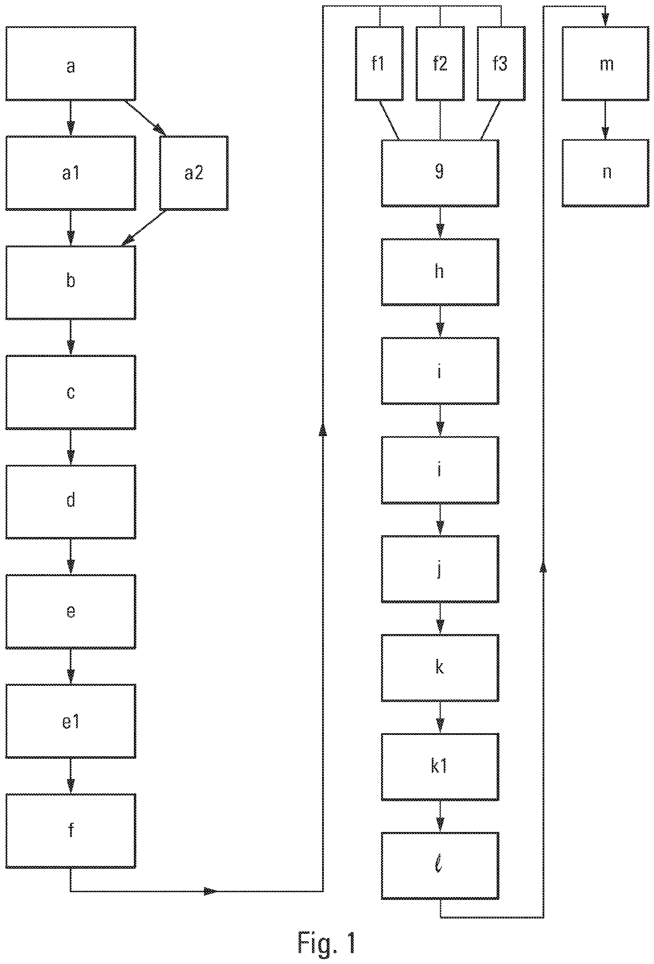

On se référera tout d'abord à la figure 1 pour décrire les différentes étapes successives du procédé de greffage d'un polymère bioactif, tel que du PolyNaSS (polystyrène sulfonate de sodium), sur des implants notamment en titane ou en alliage de titane, afin de réaliser un revêtement antiadhésif ou « anti-accroche » sur lequel les bactéries et autres agents infectieux glissent de sorte qu'ils ne peuvent pas s'y développer.

Les étapes majeures successives sont les suivantes :
a) Monter des implants sur une structure de support d'implants : on verra ci-après un élément de support pour implants dentaires et un étrier de support pour implants fémoraux de hanche.
b) Plonger les implants (montés sur leur support) dans un bain d'acide, tel que de l'acide nitrique et/ou fluorhydrique, pour les décaper : le temps de trempage peut être de l'ordre de 30 s à 1 minute.
c) Rincer les implants, par exemple à l'eau,
d) Plonger les implants (montés sur leur support) dans un bain d'anodisation, par exemple à base d'acide orthophosphorique, pour les anodiser et ainsi créer des peroxydes de titane en surface: le temps de trempage peut être de l'ordre de 10s à 1 minute,
e) Rincer les implants, par exemple à l'eau,
f) Introduire les implants (encore montés sur leur support, ou un autre support, ou sur aucun support) dans une enceinte de polymérisation étanche remplie de gaz inerte, tel que l'argon : un autre gaz inerte peut aussi être utilisé.
g) Monter les implants (montés sur leur support, ou un autre support, ou sur aucun support) sur un ascenseur installé dans l'enceinte étanche,
h) Actionner l'ascenseur pour plonger les implants dans un bain de polymérisation, par exemple de monomère, comme le styrène sulfonate de sodium (NaSS), présent dans l'enceinte,
i) Soumettre le bain de polymérisation à un catalyseur de polymérisation, par exemple thermique ou UV, pour synthétiser du polymère bioactif à la surface des implants, et ainsi obtenir un implant revêtu d'une couche de polymère greffé, par exemple du PolyNaSS,
j) Remonter l'ascenseur pour extraire les implants revêtu du bain de polymérisation,
k) Retirer les implants de l'ascenseur,
l) Extraire les implants de l'enceinte étanche,
m) Laver les implants, par exemple par aspersion d'eau pure, pour les débarrasser de l'excès de polymère bioactif non greffé,
n) Sécher les implants greffés.

Outre ces étapes majeures, le procédé définit également des étapes intermédiaires, secondaires et/ou optionnelles qui améliorent encore davantage les étapes majeures ou permettent une manipulation plus aisée d'implants particuliers, comme par exemple des implants dentaires ou encore des implants fémoraux de hanche. On peut notamment citer les étapes suivantes.

Pour les implants dentaires :
- une étape intermédiaire a1- entre l'étape a- et l'étape b-, consistant à monter plusieurs implants sur des éléments de support, eux-mêmes montés sur une platine de support, comprenant avantageusement un manche de préhension amovible, la platine avec ses éléments de support d'implants constituant une structure de support d'implants,
- les implants, lors des étapes b- à f-, sont manipulés au moyen de la platine de support, avec les éléments de support d'implants montés dessus,
- une étape intermédiaire e1- entre l'étape e- et l'étape f-, consistant à placer la platine de support avec ses éléments de support d'implants dans un récipient rempli de gaz inerte, tel que l'argon, avantageusement pourvu d'un couvercle étanche, le récipient étant ensuite introduit lors de l'étape f- dans l'enceinte étanche remplie de gaz inerte, tel que l'argon, le récipient étant alors ouvert pour en extraire la platine de support avec ses éléments de support d'implants,
- une étape intermédiaire f1- entre l'étape f- et l'étape g-, consistant à retirer les éléments de support d'implants de la platine de support, puis à monter les éléments de support d'implants sur une plaque de support qui est ensuite montée sur l'ascenseur, ou, e, variante, une étape intermédiaire f2-entre l'étape f- et l'étape g-, cette étape f2- consistant à monter sur des tiges axiales verticales (C) de l'ascenseur (L') qui sont entraînées en rotation dans le bain de polymérisation, soit la platine de support (B), soit les éléments de support (S).
- une étape intermédiaire k1- entre l'étape k- et l'étape l-, consistant à retirer les éléments de support d'implants de l'ascenseur, puis à monter les éléments de support d'implants sur une poutre centrale formant des logements de montage pour les éléments de support d'implants afin de former un panier le lavage, qui est ensuite extrait de l'enceinte. Le panier de lavage peut également être constitué à la sortie de l'enceinte étanche.
- lors des étapes m- et n-, les éléments de support d'implants sont configurés sous la forme du panier de lavage.

Pour les implants fémoraux de hanche :
- une étape intermédiaire a2- entre l'étape a- et l'étape b-, consistant à monter plusieurs implants (H) sur un étrier de support (Th), les implants (H), lors des étapes b- à f-, étant manipulés au moyen de l'étrier de support (B),
- une étape intermédiaire f3- entre l'étape f- et l'étape g-, consistant à monter les implants côte à côte sur une réglette qui est ensuite montée sur l'ascenseur,
- lors de l'étape l-, les implants sont sur la réglette,
- lors des étapes m- et n-, les implants sont disposés sur un casier de lavage.

A titre d'exemple, ce procédé de greffage de polymère actif sera maintenant mis en oeuvre sur des implants dentaires pour lesquels il a été développé des outils, accessoires ou ancillaires permettant une manipulation plus aisée, plus rapide et à grande échelle.

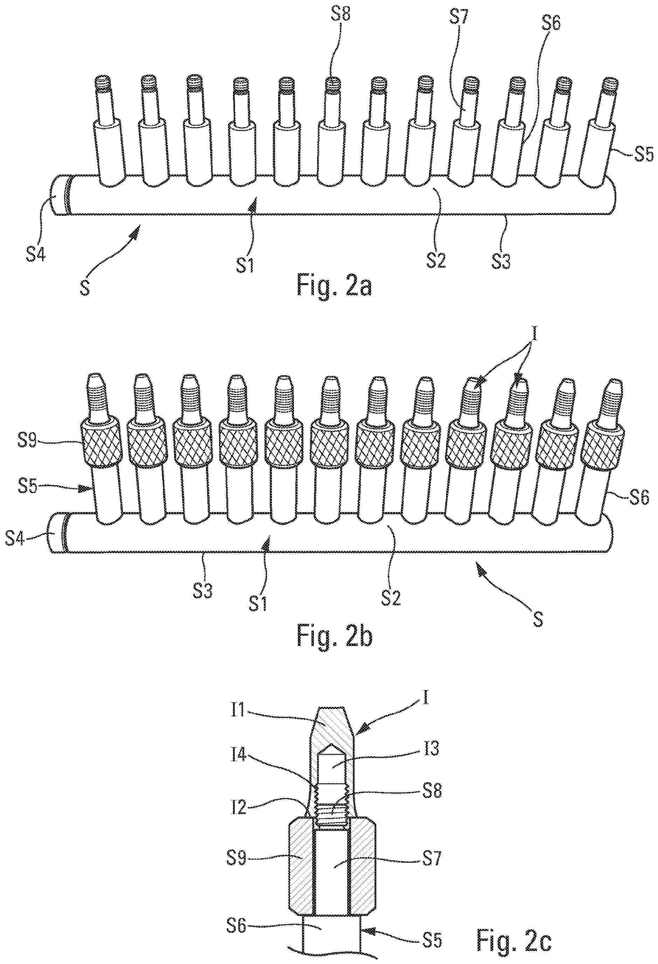

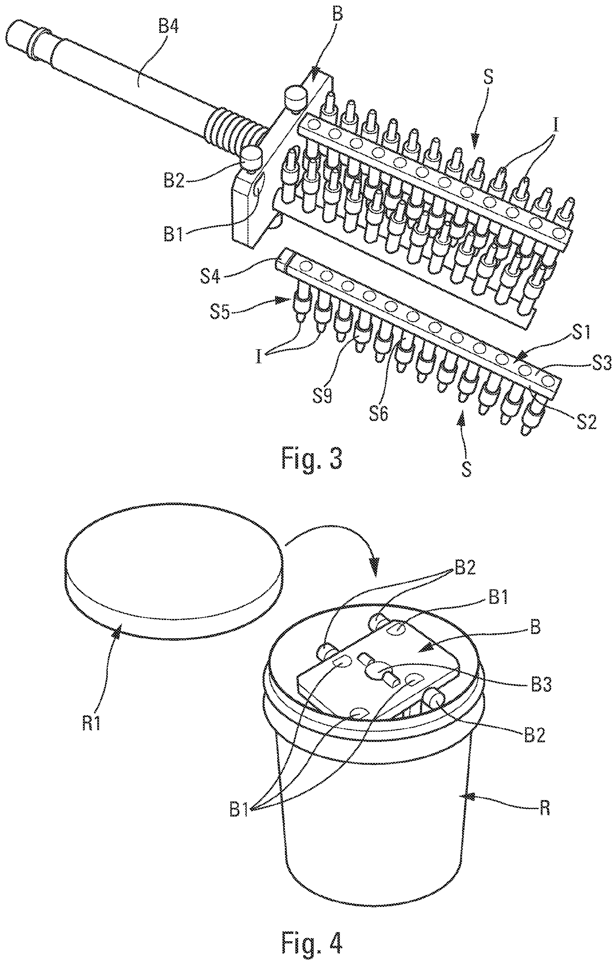

En se référant aux figures 2a, 2b et 2c, on peut voir un élément de support S pour des implants, tels que des implants dentaires I. Un type d'implants l est plus particulièrement visible en coupe sur la figure 2c. On peut voir que l'implant I comprend une tête I1 à son extrémité supérieure et un bord inférieur annulaire I2 à son extrémité opposée. L'implant I définit un intérieur creux 13 présentant une paroi filetée I4. Il s'agit là d'une conception tout à fait classique pour un implant dentaire. L'élément de support S comprend une barrette S1 sur laquelle sont montées plusieurs broches S5 qui s'étendent avantageusement parallèlement les unes aux autres. On peut par exemple prévoir douze broches S5 disposées de manière alignée et parallèle sur une barrette S1 avec un écart entre l'axe de broche de l'ordre de 1 à 2 cm. Selon un mode de réalisation avantageux, la barrette présente une section transversale cylindrique circulaire tronquée à sa base de manière à former un méplat longitudinal qui est disposé de manière opposée aux broches S5. Ceci est plus visible sur la figure 3. La barrette S1 comprend au moins une extrémité de montage pour le montage de la barrette sur une structure de support, qui sera définie ci-après. Cette extrémité de montage S4 peut même présenter un logement creux. Quant aux broches S5, elles comprennent chacune une embase S6 raccordée à la barrette S1, une tigelle S7 montée sur l'embase S5 en définissant un diamètre inférieur et comprenant une extrémité libre pourvue d'un filetage S8. Un épaulement est ainsi formé au niveau de l'extrémité supérieure de l'embase autour de la tigelle S7. Chaque broche S5 est en outre pourvue d'une bague rotative S9 qui est engagée autour de la tigelle S7 en prenant appui sur l'embase S5. L'extrémité libre filetée S8 fait saillie hors de la bague rotative S9 afin de permettre un vissage de l'implant I sur l'extrémité libre filetée en venant en butée sur la bague rotative S9, comme cela est représenté sur la figure 2c. On peut clairement voir que l'extrémité libre filetée S8 est en prise avec le filetage interne I4 du logement creux I3 de l'implant I. Il suffit que l'implant I soit à peine vissé sur l'extrémité libre filetée S8 pour garantir son maintien sur la tigelle S7 en appui sur la bague rotative S9. Les éléments de support S avec les implants I montés dessus, comme représentés sur la figure 2b, présentent une configuration globale de peigne avec des dents constituées par des broches S5 sur lesquelles sont montés les implants I.

Les bagues rotatives S9 constituent selon l'invention un système de retrait pour retirer les implants des moyens de réception de l'élément de support constitué par les extrémités libres filetées S8. En effet, en entraînant les bagues rotatives S9 en rotation libre autour des tigelles S7, il s'ensuit un desserrage et un dévissage des implants I des extrémités supérieures filetées S8, et ce, sans qu'il soit nécessaire de venir en contact avec les implants I, et notamment avec leur partie exposée. En d'autres termes, les bagues rotatives permettent le dévissage des implants sans avoir à les toucher. Il est avantageux que les bagues rotatives S9 soient parfaitement alignées de sorte qu'une action commune et simultanée est possible sur l'ensemble des bagues rotatives pour dévisser simultanément tous les implants I d'un élément de support d'implants S. On peut par exemple imaginer une baguette rectiligne qui est mise en contact appuyé de l'ensemble des bagues rotatives S9 et à laquelle on imprime un mouvement rapide de va-et-vient, permettant d'assurer le dévissage des implants I. Les implants I peuvent alors tomber par gravité dans un récipient de récupération. Il va de soi que cette opération de retrait des implants I ne se fera qu'à la fin du procédé de greffage, après l'étape n de séchage.

Sans sortir du cadre de l'invention, on peut également imaginer que les implants I soient dépourvus de filetage interne I4 et que la barrette S1 et les broches S5 soient creuses de manière à pouvoir générer une dépression qui va plaquer les implants I sur les extrémités libres des tigelles S7. La dépression peut par exemple être créée au niveau de l'extrémité de montage S4. Dans ce cas, il n'est pas nécessaire de prévoir de système de retrait, tel que les bagues rotatives S9. Toutefois, l'application d'une dépression nécessite une mise en oeuvre plus compliquée, c'est pourquoi l'utilisation de bagues rotatives S9 est une mise en oeuvre plus simple et efficace.

En se référant à la figure 3, on peut voir trois éléments de support S montés sur une platine de montage B comprenant plusieurs logements de montage B1 dans lesquels les extrémités de montage S4 des éléments de support S sont engagées et avantageusement bloquées aux moyens de vis de blocage B2. La figure 3 représente également un quatrième élément de support S prêt à être engagé dans son logement de montage B1 encore libre. Ainsi, quatre éléments de support S peuvent être montés sur une platine de montage avec une disposition mutuelle tête bêche. En effet, les deux éléments de support S les plus hauts sont disposés avec leurs broches S5 dirigées vers le bas, alors que les deux autres éléments de support S montés en bas sont disposés avec leurs broches S5 orientées vers le haut. Selon l'invention, la platine de montage B est pourvue d'un manche de préhension amovible B4 qui permet une manipulation aisée de la platine de montage B avec ses éléments de support S montés dessus.

On peut notamment utiliser la platine de montage B avec son manche amovible B4 lors des étapes a- à e- du procédé de greffage de polymère bioactif. On peut même se servir de la platine de montage B avec son manche amovible B4 pour disposer la platine de montage B avec ses éléments de support S dans le récipient rempli de gaz inerte tel que de l'argon. Ceci est visible sur la figure 4. Le manche amovible B4 doit alors être retiré laissant apparaître son logement de montage B3 au niveau de la platine B. Le récipient R peut être obturé à l'aide d'un couvercle R1 : ceci est toutefois optionnel, car l'argon est plus lourd que l'air et reste dans le récipient R même en l'absence de couvercle. Ainsi, la platine de montage B avec ses quatre éléments de support S sont introduits à l'intérieur de l'enceinte de polymérisation E qui est remplie de gaz inerte, tel que de l'argon, en étant disposé dans le récipient R. A l'intérieur de l'enceinte E, le couvercle R1 peut être retiré et la platine de montage B avec ses éléments de support S peut être extraite du récipient R.

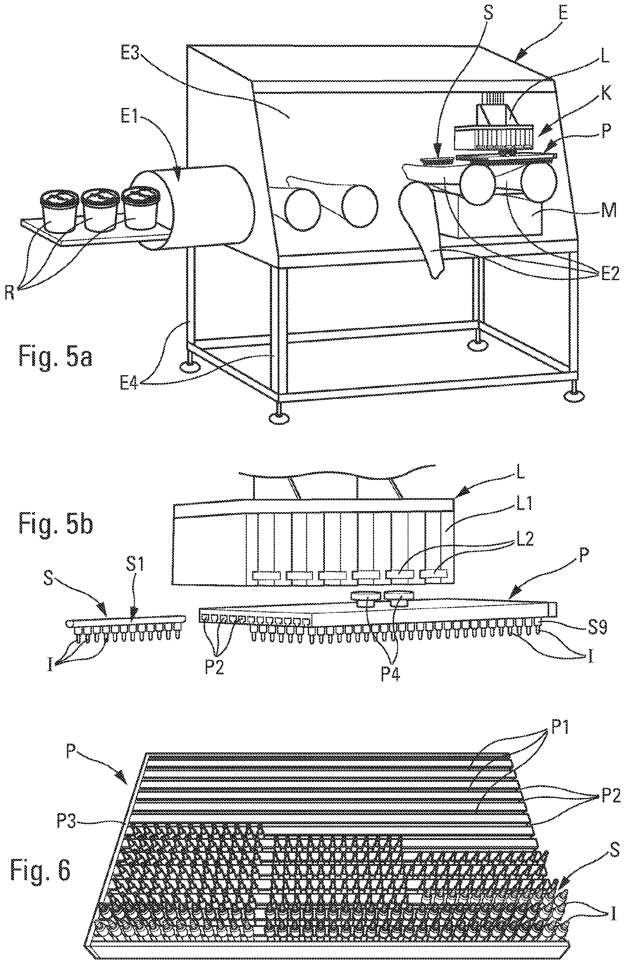

L'enceinte de polymérisation E est visible sur la figure 5a. Ce type d'enceinte est communément désigné sous l'expression « boite à gants », en raison de la présence de gants qui permettent d'effectuer des manipulations à l'intérieur de l'enceinte E à travers une paroi vitrée E3. Bien que non représentée, l'enceinte E est pourvue de moyens de traitement de son atmosphère interne afin de garantir des conditions optimales de pression, de pureté, et/ou d'hygrométrie. Le gaz le plus couramment utilisé est l'argon, bien que d'autres gaz peuvent également être mis en oeuvre. L'enceinte E est montée sur un piétement E4 et comprend un sas d'entrée E1 à travers lesquels les récipients R passent pour parvenir à l'intérieur de l'enceinte E. Le sas E1 comprend de manière conventionnelle une porte d'entrée et une porte de sortie afin de pouvoir contrôler l'atmosphère qui règne à l'intérieur du sas E1.

L'enceinte de polymérisation E contient une station de polymérisation K au niveau de laquelle les implants I sont plongés dans un bain de polymérisation pour permettre la synthèse de polymères bioactifs (par exemple du PolyNaSS) à la surface anodisée des implants, à partir d'un monomère X approprié, tel que du styrène sulfonate de sodium (NaSS). Cette station de polymérisation K comprend un ascenseur L qui est déplaçable verticalement au-dessus d'une cuve T qui est remplie de monomère X pour plonger/extraire les implants I dans et hors du bain de polymérisation de la cuve T. Cette station de polymérisation K comprend avantageusement des moyens catalyseurs pour accélérer la polymérisation sur les implants plongés dans le bain. Ces moyens catalyseurs peuvent se présenter sous la forme d'un bac de bain-marie M rempli de liquide O qui est chauffé par des moyens de chauffage M1, comme visible sur la figure 6. Les moyens catalyseurs peuvent également prendre la forme d'une source de rayonnement UV, comme on le verra ci-après.

En se référant simultanément aux figures 5b, 6 et 7 , on peut voir que l'ascenseur L comprend un chariot mobile verticalement L1 qui est monté sur une crémaillère verticale L4 de manière à permettre le déplacement vertical de haut en bas et de bas en haut du chariot L1. Ce chariot mobile est pourvu de moyens de montage aptes à recevoir une structure de support d'implants, comme on va le voir ci-après. Ces moyens de montage peuvent par exemple comprendre plusieurs glissières L2 dans lesquelles des moyens de fixation P4 de la structure de support sont insérables par coulissement. Les glissières L2 forment des ouvertures d'accès sur une face frontale tournée vers les gants de manipulation E2. L'ascenseur L peut optionnellement être pourvu d'un couvercle L3 qui vient coiffer, avantageusement de manière étanche, à la fois la cuve T remplie de monomère X et le bac de bain-marie M rempli de liquide chauffé O.

Dans le cas où le procédé de greffage de l'invention est appliqué à des implants dentaires, et plus particulièrement à des éléments de support S tels que décrits précédemment, il est prévu une plaque de montage P comprenant, sur une de ses faces, plusieurs rails de montage P1 dans lesquels les barrettes S1 des éléments de support S sont engagés par coulissement à partir d'une extrémité ouverte d'accès P2, comme cela est visible sur la figure 6 . Les éléments de support S peuvent ainsi être engagés les uns derrière les autres et les uns à côté des autres dans les rails de montage P1 de la plaque de montage P jusqu'à ce qu'elle soit pleine. Les broches S5 avec leurs implants montés dessus font saillie hors des rails de montage P1. La plaque de montage P est alors renversée et elle peut être montée dans les glissières L2 du chariot L1 au moyen d'un ou de plusieurs talon(s) de fixation P4. De cette manière, la plaque de montage P est fixée et disposée en dessous du chariot L1. L'ascenseur L peut alors être abaissé de manière à plonger les implants I dans le bain de monomères X. Le temps de polymérisation varie de 2 à 15 heures en fonction des moyens catalyseurs utilisés.

Selon un détail de manipulation, les éléments de support S sont retirés de la platine de support B à l'intérieur de l'enceinte E pour être montés individuellement dans les rails de montage P1 de la plaque de support P.

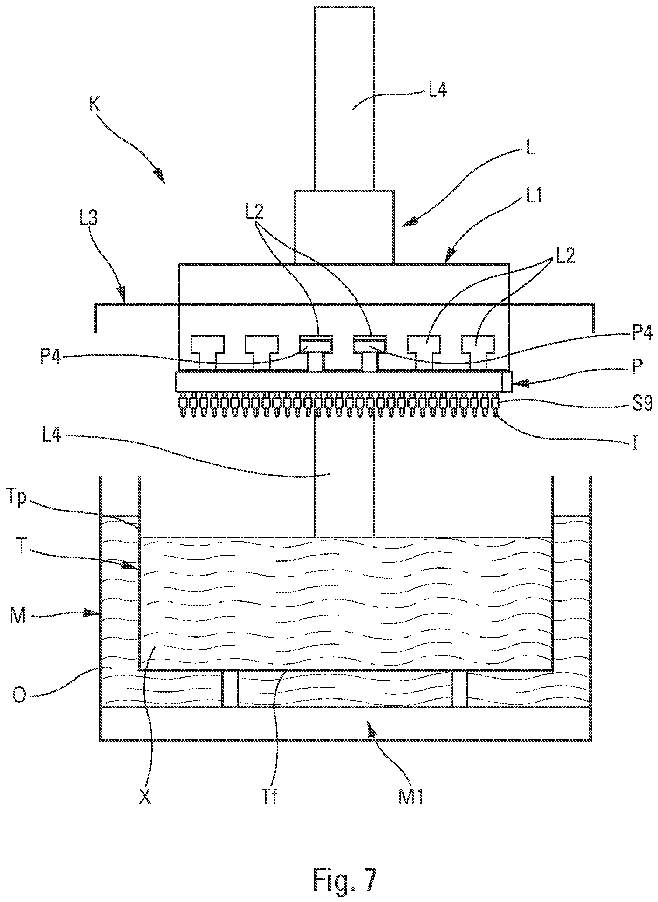

La cuve T de la figure 7, qui contient le monomère X, peut par exemple présenter une forme parallélépipédique avec un fond plat Tf et quatre parois latérales Tp. Avec des moyens catalyseurs sous la forme d'un bac de bain marie M, cette forme de réalisation est adéquate. Afin d'uniformiser la température à l'intérieur du bac de la cuve T, on peut prévoir des moyens générateurs de courant, comme par exemple un agitateur.

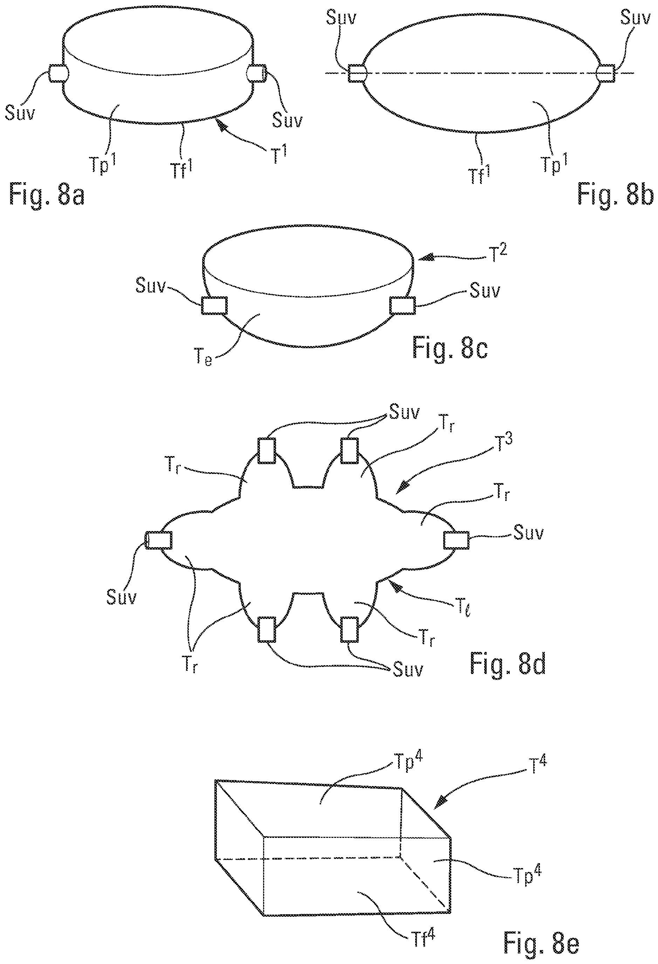

Les figures 8a à 8e illustrent de manière très schématique plusieurs variantes de réalisation pour une cuve remplie de monomère X dont les moyens catalyseurs se présentent sous la forme de sources de rayonnement UV. Sur les figures 8a et 8b, la cuve T¹ présente un fond plat Tf¹, mais une paroi latérale Tp¹ de forme elliptique. Deux sources de rayonnement UV Suv sont disposées l'une en face de l'autre sur le grand axe de l'ellipse, comme représenté sur la figure 8b. La forme elliptique de la paroi latérale Tp¹ permet d'améliorer la propagation des rayonnements UV à travers le monomère, particulièrement lorsque la paroi latérale Tp¹, et éventuellement aussi le fond Tf¹, sont réfléchissants. On peut par exemple prévoir une cuve T¹ en inox avec un fini miroir. On peut également prévoir un revêtement réfléchissement argenté à l'intérieur de la cuve T¹.

La figure 8c représente une variante dans laquelle la cuve T² présente une paroi Te en forme de coupelle ou d'auge, sans fond plat. Elle peut par exemple correspondre à une demie ellipsoïde de révolution. Les sources de rayonnement UV S_{UV} sont également disposées l'une en face de l'autre sur le grand axe de l'ellipsoïde, comme dans le mode de réalisation des figures 8a et 8b.

La figure 8d représente encore une autre variante de réalisation dans laquelle la cuve T³ est formée de plusieurs réflecteurs elliptiques Tr raccordés les uns aux autres par des tronçons de liaison Tl. La paroi latérale de la cuve T³ présente ainsi une forme complexe. Chaque réflecteur Tr est pourvu d'une source de rayonnement UV S_{UV}. La paroi latérale peut être réfléchissante, avec un fini miroir ou un revêtement argenté.

Sur la figure 8e représente une autre variante dans laquelle la cuve T⁴ est de forme parallélépipédique avec un fond plat Tf⁴ et les parois latérales Tp⁴. Une ou plusieurs de ces parois peuvent être constituées d'un panneau UV. On peut même imaginer que toute la cuve T⁴ soit constituée de cinq panneaux UV raccordés les uns autres aux autres.

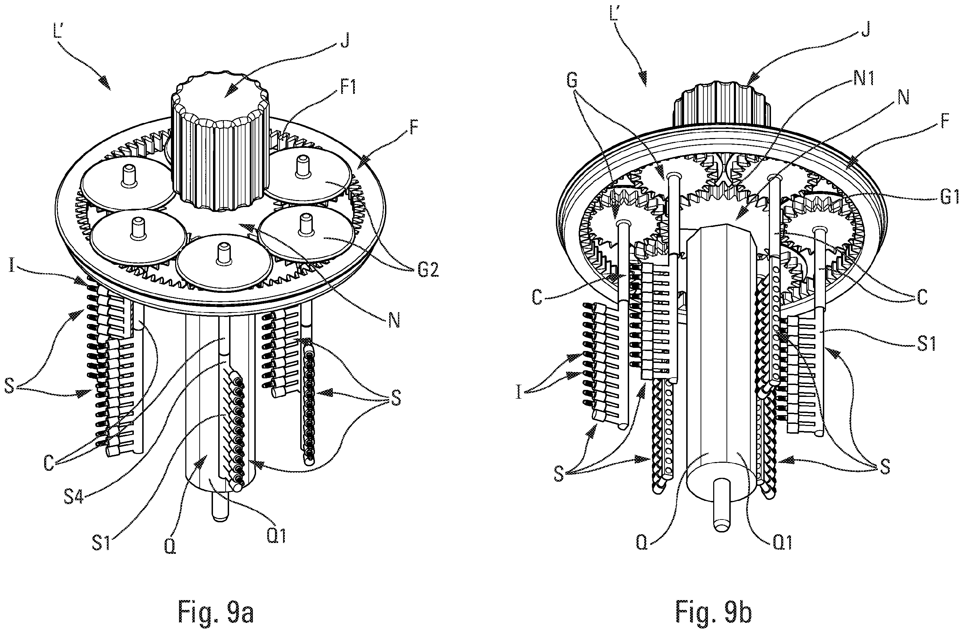

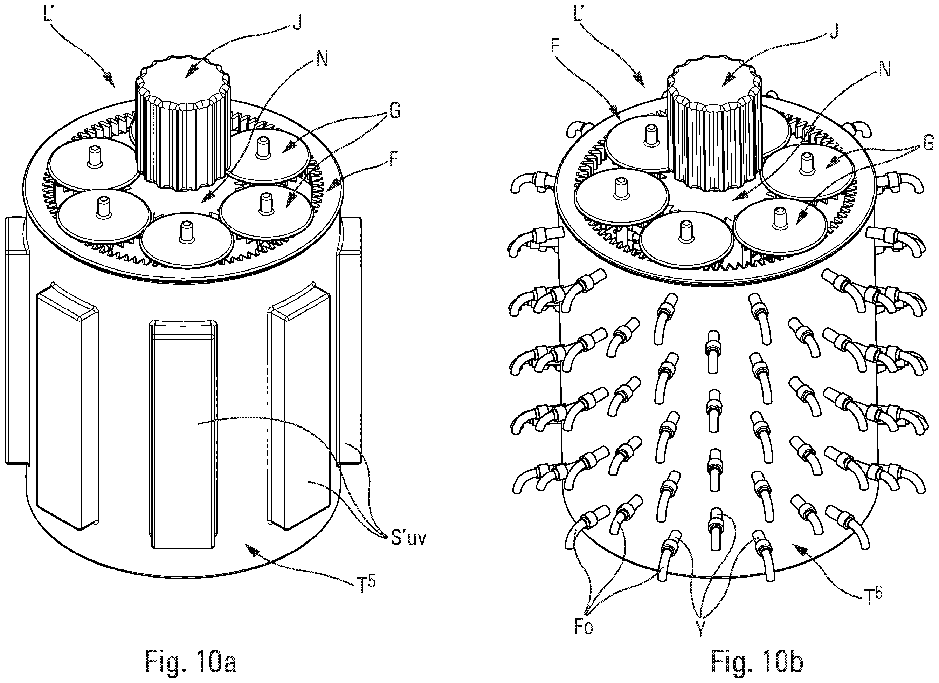

On se référera aux figures 9a, 9b, 10a et 10b pour décrire un second mode de réalisation pour un chariot L1 ' utilisable dans le cadre de la présente invention. Ce chariot L1' peut être mis en oeuvre à la place du chariot L1 précédemment décrit, qui est disposé à l'intérieur de l'enceinte étanche E. Alors que les implants I sont statiques dans la cuve lorsque le chariot L1 est dans sa position basse, les implants I montés sur le chariot L1 ' sont déplaçables à l'intérieur de la cuve, lorsque le chariot L1' est en position basse. En se référant aux figures 10a et 10b, on peut voir que les deux versions de cuves T⁵ et T⁶ présentent une forme sensiblement cylindrique avec des sources de rayonnement UV réparties sur sa périphérie. La cuve T⁵ comprend ainsi huit sources UV S'uv qui sont réparties de manière équidistante tout autour de la cuve. Les sources S'uv peuvent présenter une étendue axiale importante. Quant à la cuve T⁶, elle est pourvue d'une pluralité d'embouts optiques Y auxquels sont raccordées des fibres optiques Fo reliées à une source UV (non représentée). Compte tenu du nombre important d'embouts Y et de fibres optiques Fo, l'intérieur de la cuve T⁶ est irradiée de manière parfaitement homogène. La paroi interne de ces cuves T⁵ et T⁶ peuvent être réfléchissantes pour le rayonnement UV.

Sur les figures 10a et 10b, le chariot L1' est représenté dans sa position basse, dans lequel il est introduit au maximum à l'intérieur des cuves T⁵ et T⁶. Sur ces figures, il n'est pas représenté de quelle manière le chariot L1' est déplacé axialement verticalement, mais n'importe quel moyen approprié peut être utilisé.

On se référera maintenant aux figures 9a et 9b pour décrire en détail la structure et le fonctionnement de ce chariot L1'. Il est ici destiné à recevoir des éléments de support S tels que décrits précédemment, mais il peut également être envisageable que des platines de support B dotées de plusieurs éléments de support S soient reçus par le chariot L1'. Dans le cas particulier des figures 9a et 9b, chaque élément de support S est monté par son extrémité de montage S4 à l'extrémité libre d'une tige axiale verticale C qui forme l'axe de rotation d'une roue horizontale G. Chaque roue G est dotée d'un disque supérieur G1 à travers lequel débouche l'extrémité supérieure de la tige axiale verticale C. Sur les figures 9a et 9b, les roues horizontales G sont en nombre de six, mais ce nombre n'est pas limitatif. Ces roues horizontales G sont disposées à l'intérieur d'une couronne de révolution F qui est dentée intérieurement en F1. Les dents G1 des roues horizontales G sont en prise avec les dents F1 de la couronne de révolution F. Le disque G2 repose avec leur bord externe sur la couronne de révolution F. D'autre part, les roues dentées G sont également en prise avec une roue d'entraînement centrale N qui est également dentée en N1. Cette roue centrale N est pourvue en partie haute d'un plot d'entraînement J destiné à venir en prise avec des moyens d'entraînement en rotation (non représentés). A son extrémité inférieure, la roue d'entraînement N est pourvue d'un moyeu Q qui s'étend entre les tiges axiales verticales C et les éléments de support S. Avantageusement, le moyeu Q est réfléchissant, tout particulièrement aux rayonnements UV. On peut par exemple réaliser le moyeu Q avec des facettes réfléchissantes Q1. Bien que non représenté, le chariot L1' peut être pourvu d'un couvercle qui coiffe hermétiquement les roues horizontales G et la couronne de révolution F.

Avec une telle conception, on comprend aisément que l'entraînement en rotation du plot d'entraînement J a pour effet d'entraîner la roue d'entraînement N en rotation sur elle-même. Etant donné que la roue d'entraînement N est en prise engrenée avec les roues horizontales G, ces dernières sont entraînées en rotation à la fois sur elle-même, mais également autour de la roue d'entraînement N à l'intérieur de la couronne de révolution F. Par conséquent, les éléments de support S sont entraînés à la fois en rotation autour d'un axe passant par les tiges C et leurs barrettes S1, mais également en rotation de révolution autour d'un axe central passant par le plot d'entraînement J, la roue d'entraînement N et le moyeu Q. Les éléments de support S effectuent donc un mouvement complexe résultant de la combinaison d'une rotation autour de leurs barrettes et d'une révolution autour de la roue d'entraînement centrale N. De cette manière, les implants I montés sur les éléments de support S se déplacent selon une trajectoire complexe à l'intérieur du monomère présent dans la cuve. On garantit ainsi que les implants I sont exposés de manière identique et homogène au rayonnement UV qui irradie le monomère présent dans la cuve.

Avec un ascenseur L' associé à une cuve irradiée par un rayonnement UV, comme les cuves T⁵ et T⁶, on obtient un revêtement greffé de polymère bioactif, tel que du PolyNaSS, avec une épaisseur et une densité souhaitées. Le temps nécessaire pour une polymérisation satisfaisante peut être considérablement réduit par rapport à une polymérisation avec catalyseur thermique (du type bain-marie) qui est de l'ordre de 15 heures. On peut en effet réduire aisément ce temps de moitié, et même davantage avec un rayonnement UV et une cuve optimisés, pour atteindre un temps de l'ordre de 2 à 5 heures, voire encore moins de l'ordre de 1 heure.

Une fois le greffage achevé, les implants sont extraits de la cuve en remontant le chariot L1 ou L1 '. Les éléments de support S peuvent alors être retirés du chariot en les manipulant à l'aide des gants de manipulation E2. Ils peuvent être ainsi amenés dans le sas d'entrée E1, d'où ils sont extraits de l'enceinte étanche E. Ils peuvent ensuite subir les étapes de lavage pour les débarrasser de l'excès de polymère bioactif greffé et de séchage.

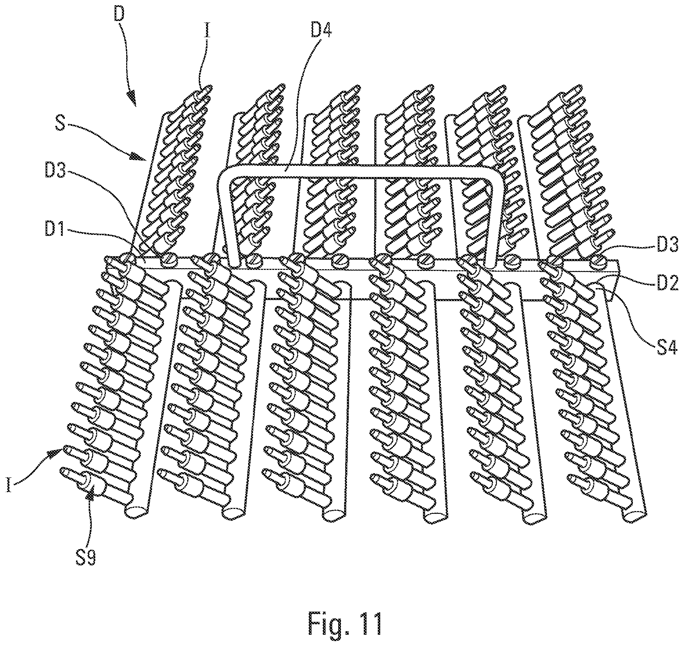

En se référant à la figure 11, on peut voir un autre ustensile ou ancillaire qui permet une manipulation avantageuse des éléments de support S lors des étapes de lavage et de séchage. Cet ustensile peut être qualifié de panier de lavage D, étant donné qu'il présente une configuration proche de celle des paniers que l'on rencontre dans les lave-vaisselles. Ce panier de lavage D comprend une poutre centrale D1 qui forme plusieurs logements de montage D2 dans lesquels sont engagées les extrémités de montage S4 des éléments de support S. Des vis de blocage optionnels D3 peuvent être prévus pour bloquer les extrémités de montage S4 à l'intérieur des logements de montage D2. La poutre centrale D1 peut également être pourvue d'une anse de préhension D4 par laquelle on peut saisir manuellement le panier de lavage D. Sur la figure 11, on peut dénombrer douze éléments de support S, à savoir six de chaque côté de la poutre centrale D1. On peut remarquer que les éléments de support S sont disposés avec une orientation inclinée, de l'ordre de 45° par rapport à l'horizontale ou la verticale. L'inclinaison des éléments de support S de part et d'autre de la poutre centrale D1 sont opposés, ou décalés de 90°. En effet, les implants I visibles en dessous de la poutre centrale D1 sur la figure 11 sont orientés vers la gauche, alors que les implants I disposés au-dessus de la poutre centrale D1 sont orientés vers la droite.

Ce panier de lavage D peut ainsi aisément être disposé dans un équipement de lavage approprié, dans lequel de l'eau, de préférence hautement purifiée, est projetée par aspersion sur les implants I, afin de les débarrasser de leur excès de polymère bioactif greffé. Cette phase de lavage est suivie par une phase de séchage qui peut être opérée dans le même appareil de lavage.

A la fin de l'étape de séchage, le panier D est extrait de l'appareil de lavage/séchage, et les éléments de support S sont retirés de la poutre centrale D1. Ensuite, les implants I peuvent être retirés de l'élément de support S, comme précédemment expliqué, en entraînant les bagues rotatives S9 en rotation, afin de dévisser les implants I des extrémités filetées S8. Sans sortir du cadre de l'invention, on peut également imaginer que les implants I soient maintenus par aspiration sur des éléments de support appropriés.

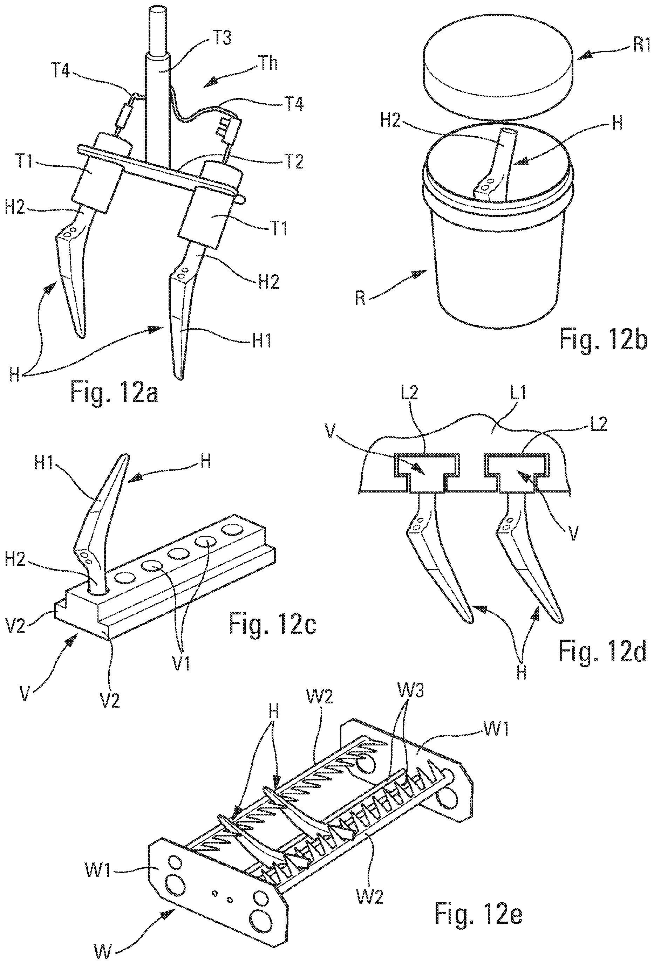

Jusqu'à présent, toute la description a été faite en référence à un type d'implant particulier, à savoir les implants dentaires I. L'invention n'est toutefois pas limitée à ce type d'implant particulier, et d'autres types d'implants peuvent être traités, revêtus et manipulés selon l'invention. En se référant aux figures 12a à 12f, il est fait référence à un autre type d'implant, à savoir un implant fémorale de hanche H. On peut également parler de prothèse fémorale. Cet insert H comprend de manière typique une broche fémorale H1 destinée à être insérée et scellée dans le fémur et une tige de col H2 destinée à recevoir la tête fémorale qui est reçue dans la cotyle fixé au bassin. Les prothèses fémorales H ici traitées sont dépourvues de têtes.

En se référant à la figure 12a, on peut voir un premier dispositif de support sous la forme d'un étrier Th qui permet de recevoir deux implants fémoraux H. Plus précisément, cet étrier Th comprend deux manchons de réception T1 dans lesquels sont reçues respectivement les tiges de col H2 des implants H. Ces manchons T1 sont montés sur une plaquette commune qui est pourvue d'une tige de montage T3. Les deux manchons T1 sont également pourvus de moyens de câblage électriques T4 pour alimenter les inserts H en courant. Cet étrier Th peut être utilisé lors des étapes b, c, d, et e du procédé de greffage. Cet étrier Th constitue une structure de support d'implants, au même titre que l'élément de support S, la platine de support B et la plaque de support précédemment décrits. L'étrier Th peut être monté sur un ascenseur au moyen de sa barre de montage T3 pour réaliser les étapes de décapage, d'anodisation et de rinçage.

Une fois ces étapes terminées, les implants H peuvent être retirés de l'étrier Th et placés individuellement ou en groupe dans un récipient R, tel que celui utilisé précédemment pour les implants dentaires. Ce récipient R est avantageusement rempli d'un gaz inerte, tel que de l'argon, et pourvu optionnellement d'un couvercle R1. Les implants peuvent ainsi être introduits dans l'enceinte de polymérisation E à travers le sas d'entrée E1. A l'intérieur de l'enceinte, les implants H peuvent être montés sur une réglette V, visible sur la figure 12c. Cette réglette V comprend des trous de réception V1 dans lesquels les tiges de col H2 sont insérées en force. La réglette V comprend également deux brides longitudinales opposées V2, qui vont permettre l'insertion par coulissement des réglettes V dans les glissières L2 du chariot L1 de l'ascenseur L, comme visible sur la figure 12d. Les implants H peuvent ainsi être plongés dans la cuve remplie de monomère X. Après un temps de polymérisation suffisant, les implants peuvent être extraits de la cuve et les réglettes V peuvent être extraites des glissières L2. Les implants H peuvent ensuite être retirés des réglettes V et extraits de l'enceinte étanche E à travers le sas d'entrée. En variante, les implants H peuvent être montés sur les tiges axiales verticales C de l'ascenseur L'. Il est également envisageable de monter plusieurs implants H sur une même tige axiale verticale C en utilisant une structure de support, tel que l'étrier Th.

Pour les étapes de lavage et de séchage ultérieures, il est prévu un casier de lavage W (figure 12e) sur lequel les implants greffés peuvent être déposés. Ce casier de lavage W comprend deux flasques d'extrémité W1 reliées entre elles par deux râteaux de rangement W2 et deux tiges de support W3. Les implants H peuvent ainsi être disposés entre les dents des râteaux W2 et en appui sur les tiges de support W3.

Il est à noter que certains aspects particuliers liés à la manipulation des implants fémoraux H peuvent être protégés indépendamment des autres caractéristiques déjà exposées et pourraient de ce fait faire l'objet de demandes divisionnaires. Plus particulièrement, l'étrier Th, la réglette V et le casier de lavage W pourraient faire l'objet de dépôts de brevet en soi.

Grâce à l'invention, on dispose d'un procédé de greffage de polymère bioactif sur des implants qui peut mis en oeuvre de manière industrielle à très grande échelle. Les ustensiles qui ont été conçus, tels que l'élément de support S, la platine de support B, la plaque de support P, le casier de lavage D, l'étrier de support Th, la réglette V et le casier de lavage W permettent d'optimiser le procédé de greffage sur le plan industriel. Enfin, l'enceinte étanche E, et plus particulièrement son chariot et sa cuve rendent possibles une industrialisation à grande échelle de l'étape de polymérisation. Des implants, notamment dentaires et de hanche, peuvent ainsi être revêtus avec un polymère bioactif, tel que du PolyNaSS, à grande échelle et à grande cadence.

## Revendications

1. Enceinte étanche (E) contenant un gaz inerte, tel que de l'argon, l'enceinte étanche (E) comprenant un sas d'entrée (E1), **caractérisée en ce qu'**elle contient en outre une station de polymérisation (K) pour greffer un polymère bioactif, tel que du PolyNaSS, sur un implant (I ; H), la station de polymérisation (K) comprenant :
- une cuve (T ;T¹ ; T², T³ ; T⁴ ; T⁵ ; T⁶) destinée à contenir un monomère (X),
- des moyens catalyseurs (M ; S_{UV} ; S'_{UV}), à savoir un bac de bain-marie (M) dans lequel est disposée la cuve (T) ou au moins une source de rayonnements UV (S_{UV} ; S'_{UV}) qui irradient le contenu de la cuve (T¹ ; T², T³ ; T⁴ ; T⁵ ; T⁶), agissant sur le monomère (X) de la cuve (T ;T¹ ; T², T³ ; T⁴ ; T⁵ ; T⁶) pour accélérer la polymérisation,
- un ascenseur (L) comprenant un chariot (L1 ; L1') déplaçable verticalement au-dessus de la cuve, le chariot (L1 ; L1 ') étant pourvu de moyens de montage (L2 ; C) aptes à recevoir des implants (H) ou une structure de support (P) d'implants (I ; H) pour les plonger/extraire dans/hors de la cuve (T ;T¹ ; T², T³ ; T⁴ ; T⁵ ; T⁶).

2. Enceinte étanche (E) selon la revendication 1, dans laquelle la cuve (T¹ ; T², T³ ; T⁵; T⁶) comprend une paroi de fond (Tf¹) et au moins une paroi latérale (Tp¹; Te ; Tr), au moins une paroi parmi la paroi de fond (Tf¹) et ladite au moins une paroi latérale (Tp¹; Te; Tr) est réfléchissante pour le rayonnement UV.

3. Enceinte étanche (E) selon la revendication 1 ou 2, dans laquelle la cuve (T¹ ; T², T³ ; T⁵ ; T⁶) comprend une paroi de fond (Tf¹) et au moins une paroi latérale (Tp¹; Te ; Tr), au moins une paroi parmi la paroi de fond (Tf¹) et ladite au moins une paroi latérale (Tp¹; Te ; Tr) présente une forme elliptique.

4. Enceinte étanche (E) selon la revendication 1, 2 ou 3, dans laquelle la cuve comprend une paroi de fond et une paroi latérale elliptique pourvue d'au moins deux sources de rayonnement UV disposées sur le grand axe de l'ellipse.

5. Enceinte étanche (E) selon la revendication 1, 2 ou 3, dans laquelle la cuve (T³) comprend une paroi latérale formant plusieurs cavités de réflexion (Tr), avantageusement elliptiques, pourvues chacune d'une source de rayonnement UV (S_{UV}).

6. Enceinte étanche (E) selon l'une quelconque des revendications précédentes, dans laquelle la cuve (T4) comprend une paroi de fond (Tf⁴) et au moins une paroi latérale (Tp⁴), au moins une paroi parmi la paroi de fond (Tf⁴) et ladite au moins une paroi latérale (Tp⁴) comprend un panneau LED UV.

7. Enceinte étanche (E) selon l'une quelconque des revendications précédentes, dans laquelle la cuve (T4) comprend une paroi de fond (Tf⁴) et au moins une paroi latérale (Tp⁴), au moins une paroi parmi la paroi de fond (Tf⁴) et ladite au moins une paroi latérale (Tp⁴) est formée d'un panneau LED UV.

8. Enceinte étanche (E) selon l'une quelconque des revendications précédentes dans laquelle les moyens de montage (L2) comprennent au moins une glissière dans laquelle des moyens de fixation (P4) de la structure de support (P) sont insérables par coulissement.

9. Enceinte étanche (E) selon l'une quelconque des revendications précédentes, dans laquelle l'ascenseur (L) comprend un couvercle (L3) mobile verticalement pour couvrir la cuve (T ;T¹ ; T², T³ ; T⁴ ; T⁵ ; T⁶) en position basse.

10. Enceinte étanche (E) selon l'une quelconque des revendications 1 à 9, dans laquelle le chariot (L1') comprend au moins une roue horizontale (G) qui est montée rotative autour d'une tige axiale verticale (C) sur laquelle est monté l'implant ou une structure de support d'implants, de sorte que l'implant tourne sur la tige axiale verticale (C) dans le monomère de la cuve (T⁵ ; T⁶).

11. Enceinte étanche (E) selon la revendication 10, dans laquelle la roue horizontale (G) se déplace selon une trajectoire circulaire à l'intérieur d'une couronne de révolution (F) autour une roue d'entrainement centrale (N), de sorte que l'implant tourne sur la tige axiale verticale (C) qui se déplace selon la trajectoire circulaire dans le monomère de la cuve (T⁵ ; T⁶).

12. Enceinte étanche (E) selon la revendication 10 ou 11, dans laquelle la roue d'entrainement centrale (N) est pourvue d'un moyeu réfléchissant (Q) qui plonge dans la cuve (T⁵ ; T⁶).

## Patentansprüche

1. Abgedichtete Kammer (E), die mit einem Inertgas, beispielsweise Argon, gefüllt ist, wobei die abgedichtete Kammer (E) eine Einführschleuse (E1) aufweist, **dadurch gekennzeichnet, dass** die Kammer ferner eine Polymerisationsstation (K) enthält, in der ein bioaktives Polymeres, beispielsweise PolyNaSS, auf ein Implantat (I; H) gepfropft wird, wobei die Polymerisationsstation (K) folgendes umfasst:
- einen Behälter (T; T¹; T², T³; T⁴; T⁵; T⁶), in dem ein Monomeres (X) enthalten ist,
- Katalysatormittel (M; Suv; S'uv), nämlich eine Wasserbad-Wanne (M), in der der Behälter (T) oder mindestens eine Quelle (Suv; S'_{UV}) für UV-Strahlung angeordnet ist, mit der der Inhalt des Behälters (T; T¹; T², T³; T⁴; T⁵; T⁶) bestrahlt wird, wobei sie auf das Monomere (X) im Behälter (T; T¹; T², T³; T⁴; T⁵; T⁶) einwirkt, um die Polymerisation zu beschleunigen,
- einen Aufzug (L) mit einem Schlitten (L1; L1'), der vertikal über dem Behälter verfahrbar ist, wobei der Schlitten (L1; L1') mit Befestigungsmitteln (L2; C) versehen ist, die zur Aufnahme von Implantaten (H) oder einer Trägerstruktur (P) für Implantate (I; H) ausgelegt sind, welche dann in den Behälter (T; T¹; T², T³; T⁴; T⁵; T⁶) eingetaucht bzw. aus diesem wieder herausgezogen werden.

2. Abgedichtete Kammer (E) nach Anspruch 1, bei der der Behälter (T¹; T², T³; T⁵; T⁶) eine Bodenwand (Tf¹) und mindestens eine Seitenwand (Tp¹; Te; Tr) umfasst, wobei mindestens eine dieser Wände, also die Bodenwand (Tf¹) oder die mindestens eine Seitenwand (Tp¹; Te; Tr), UV-Strahlung reflektiert.

3. Abgedichtete Kammer (E) nach einem der Ansprüche 1 oder 2, bei der der Behälter (T¹; T², T³; T⁵; T⁶) eine Bodenwand (Tf¹) und mindestens eine Seitenwand (Tp¹; Te; Tr) aufweist, wobei mindestens eine dieser Wände, also die Bodenwand (Tf¹) oder die mindestens eine Seitenwand (Tp¹; Te; Tr), eine elliptische Form aufweist.

4. Abgedichtete Kammer (E) nach einem der Ansprüche 1, 2 oder 3, bei der der Behälter eine Bodenwand und eine ellipsenförmige Seitenwand umfasst, die mit mindestens zwei Quellen für UV-Strahlung versehen ist, die auf der Hauptachse der Ellipse angeordnet sind.

5. Abgedichtete Kammer (E) nach einem der Ansprüche 1, 2 oder 3, bei der der Behälter (T³) eine Seitenwand umfasst, in der mehrere, vorzugsweise ellipsenförmige Reflexionsausnehmungen (Tr) ausgebildet sind, in denen jeweils eine Quelle (Suv) für UV-Strahlung angeordnet ist.

6. Abgedichtete Kammer (E) nach einem der voranstehenden Ansprüche, bei der der Behälter (T4) eine Bodenwand (Tf⁴) und mindestens eine Seitenwand (Tp⁴) aufweist, wobei mindestens eine dieser Wände, also die Bodenwand (Tf⁴) oder die mindestens eine Seitenwand (Tp⁴), ein UV-LED-Panel beinhaltet.

7. Abgedichtete Kammer (E) nach einem der voranstehenden Ansprüche, bei der der Behälter (T4) eine Bodenwand (Tf⁴) und mindestens eine Seitenwand (Tp⁴) aufweist, wobei mindestens eine dieser Wände, also die Bodenwand (Tf⁴) oder die mindestens eine Seitenwand (Tp⁴), aus einem UV-LED-Panel besteht.

8. Abgedichtete Kammer (E) nach einem der voranstehenden Ansprüche, bei der die Befestigungsmittel (L2) mindestens eine Führung umfassen, in die die Mittel (P4) zur Befestigung der Trägerstruktur (P) eingeschoben werden können.

9. Abgedichtete Kammer (E) nach einem der voranstehenden Ansprüche, bei der der Aufzug (L) eine vertikal bewegliche Abdeckung (L3) umfasst, mit der der Behälter (T; T¹; T², T³; T⁴; T⁵; T⁶) in der abgesenkten Stellung abgedeckt wird.

10. Abgedichtete Kammer (E) nach einem der Ansprüche 1 bis 9, bei der der Schlitten (L1') mindestens folgendes umfasst:
ein horizontales Zahnrad (G), das drehbar an einer vertikalen axialen Stange (C), an der das Implantat oder eine Implantatträgerstruktur angebracht ist, gelagert ist, so dass sich das Implantat an der vertikalen axialen Stange (C) in dem im Behälter (T⁵; T⁶) enthaltenen Monomeren dreht.

11. Abgedichtete Kammer (E) nach Anspruch 10, bei der sich das horizontale Zahnrad (G) in einer Kreisbahn innerhalb eines Drehkranzes (F) um ein zentrales Antriebsrad (N) herum derart bewegt, dass sich das Implantat an der vertikalen axialen Stange (C) dreht, die sich entsprechend der Kreisbahn in dem im Behälter (T⁵; T⁶) enthaltenen Monomeren bewegt.

12. Abgedichtete Kammer (E) nach Anspruch 10 oder 11, bei der das zentrale Antriebsrad (N) mit einer reflektierenden Nabe (Q) versehen ist, die in den Behälter (T⁵; T⁶) eintaucht.

## Claims

1. A gastight chamber (E) containing an inert gas, such as argon, the gastight chamber (E) including an inlet air lock (E1), the gastight chamber being **characterized in that** it further contains a polymerization station (K) for grafting a bioactive polymer, such as PolyNaSS, on an implant (I; H), the polymerization station (K) comprising:
- a vessel (T; T¹; T², T³; T⁴; T⁵; T⁶) for containing a monomer (X);
- catalyst means (M; Suv; S'uv) a "bain-marie" (M) in which the vessel (T) is placed or at least one UV radiation source (Suv; S'uv) that irradiates the contents of the vessel (T¹; T², T³; T⁴; T⁵; T⁶). that act on the monomer (X) of the vessel (T; T¹; T², T³; T⁴; T⁵; T⁶) so as to accelerate polymerization;
- an elevator (L) including a carriage (L1; L1') that is movable vertically above the vesse the carriage (L1; L1') that is provided with mounting means (L2; C) that are suitable for receiving implants (H) or a support structure (P) for supporting implants (I; H). so as to dip and extract them into and therefrom.the vessel (T; T¹; T², T³; T⁴; T⁵; T⁶)

2. A gastight chamber (E) according to claim 1 wherein the vessel (T¹; T², T³; T⁵; T⁶) comprises a bottom wall (Tf¹) and at least one side wall (Tp¹; Te; Tr), at least one wall from among the bottom wall (Tf¹) and said at least one side wall (Tp¹; Te; Tr) is reflective for UV radiation.

3. A gastight chamber (E) according to claim 1 or claim 2, wherein the vessel (T¹; T², T³; T⁵; T⁶) comprises a bottom wall (Tf¹) and at least one side wall (Tp¹; Te; Tr), at least one wall from among the bottom wall (Tf¹) and said at least one side wall (Tp¹; Te; Tr) presents a shape that is elliptical.

4. A gastight chamber (E) according to claim 1, 2, or 3, wherein the vessel comprises a bottom wall and an elliptical side wall that is provided with at least two UV radiation sources arranged on the major axis of the ellipse.

5. A gastight chamber (E) according to claim 3, 4, or 5, wherein the vessel (T³) comprises a side wall that forms a plurality of reflective cavities (Tr) that are advantageously elliptical and that are each provided with a UV radiation source (S_{UV}).

6. A gastight chamber (E) according to any preceding claim, wherein the vessel (T4) comprises a bottom wall (Tf⁴) and at least one side wall (Tp⁴), at least one wall from among the bottom wall (Tf⁴) and said at least one side wall (Tp⁴) includes a UV LED panel.

7. A gastight chamber (E) according to any preceding claim, wherein the vessel (T4) comprises a bottom wall (Tf⁴) and at least one side wall (Tp⁴), at least one wall from among the bottom wall (Tf⁴) and said at least one side wall (Tp⁴) is formed by a UV LED panel.

8. A gastight chamber (E) according to any preceding claim wherein the mounting means (L2) include at least one slideway into which fastener means (P4) of the support structure (P) can be inserted by sliding.

9. A gastight chamber (E) according to any preceding claim, wherein the elevator (L) includes a lid (L3) that is movable vertically so as to cover the vessel (T; T¹; T², T³; T⁴; T⁵; T⁶) when the elevator is in its low position.

10. A gastight chamber (E) according to any one of claims 1 to 9, wherein the carriage (L1') includes at least one horizontal wheel (G) that is mounted to turn about a vertical axial rod (C) on which the implant or an implant support structure is mounted, such that the implant turns on the vertical axial rod (C) in the monomer of the vessel (T⁵; T⁶).

11. A gastight chamber (E) according to claim 10, wherein the horizontal wheel (G) moves along a circular path inside a circularly-cylindrical collar (F) about a central drive wheel (N), such that the implant turns about the vertical axial rod (C) that moves along the circular path, in the monomer of the vessel (T⁵; T⁶).

12. A gastight chamber (E) according to claim 10 or claim 11, wherein the central drive wheel (N) is provided with a reflective hub (Q) that dips into the vessel (T⁵; T⁶).
